(19) Europäisches Patentamt
European Patent Office
Office européen des brevets

(11) EP 2 854 783 B1

(12) EUROPEAN PATENT SPECIFICATION

(45) Date of publication and mention
of the grant of the patent:
14.02.2018 Bulletin 2018/07

(51) Int Cl.:
A61K 31/05 (2006.01)          A61K 31/231 (2006.01)
A61K 31/7008 (2006.01)        A61K 8/06 (2006.01)
A61K 9/06 (2006.01)           A61P 17/00 (2006.01)
A61P 17/18 (2006.01)          A61K 8/34 (2006.01)
A61K 8/37 (2006.01)           A61K 8/60 (2006.01)
A61Q 19/02 (2006.01)          A61K 47/14 (2017.01)
A61K 9/107 (2006.01)          A61K 9/00 (2006.01)

(21) Application number: 13739805.3

(22) Date of filing: 04.06.2013

(86) International application number:
PCT/IB2013/054606

(87) International publication number:
WO 2013/182996 (12.12.2013 Gazette 2013/50)

(54) MIXTURE FOR THE INHIBITION OF MELANIN BIOSYNTHESIS

MISCHUNG ZUR HEMMUNG DER MELANINBIOSYNTHESE

MÉLANGE POUR L'INHIBITION DE LA BIOSYNTHÈSE DE LA MÉLANINE

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 04.06.2012 IT BS20120092

(43) Date of publication of application:
08.04.2015 Bulletin 2015/15

(73) Proprietor: General Topics S.R.L.
25087 Salò (Brescia) (IT)

(72) Inventor: DE PAOLI AMBROSI, Gianfranco
25087 Salò (BS) (IT)

(74) Representative: Sangiacomo, Ines
Biesse S.r.l.
Via Corfù, 71
25124 Brescia (IT)

(56) References cited:
WO-A2-2009/105294        WO-A2-2010/078985
US-A1- 2007 098 655

• G Vielhaber ET AL:
"4-(1-Phenylethyl)1,3-Benzenediol: A New,
Highly Efficient Lightening Agent", International
Journal of Cosmetic Science, 2007, 29, 1 January
2007 (2007-01-01), pages 63-66, XP055052000,
Retrieved from the Internet:
URL:http://onlinelibrary.wiley.com/store/1
0.1111/j.1467-2494.2007.00355_6.x/asset/j.
1467-2494.2007.00355_6.x.pdf?v=1&t=hcn36y4
z&s=668fef297fb66ef8f077d9262534c6eb6b2979
c9 [retrieved on 2013-02-01]
• Jae Sung Hwang ET AL: "Disruption of tyrosinase
glycosylation by N-acetylglucosamine and its
depigmenting effects in guinea pig skin and in
human skin", Journal of Dermatological Science
63 (2011), 1 September 2011 (2011-09-01), pages
199-205, XP055052115, Retrieved from the
Internet:
URL:http://ac.els-cdn.com/S092318111100176
9/1-s2.0-S0923181111001769-main.pdf?_tid=1
62c13ce-6c8b-11e2-92f2-00000aab0f27&acdnat
=1359735720_6f6f568581896de2f81318a71b0606
79 [retrieved on 2013-02-01]

- Donald L Bissett ET AL: "Reduction in the appearance of facial hyperpigmentation by topical N-acetyl glucosamine", Journal of Cosmetic Dermatology, 6, 2007, 1 March 2007 (2007-03-01), pages 20-26, XP055052117, Retrieved from the Internet: URL:http://onlinelibrary.wiley.com/store/10.1111/j.1473-2165.2007.00295.x/asset/j.1473-2165.2007.00295.x.pdf?v=1&t=hcnjrjtc&s=def179a0531d8e9c00ddc1902b84348fdd191822 [retrieved on 2013-02-01]
- Sungran Huh ET AL: "Melanogenesis Inhibitory Effect of Fatty Acid Alkyl Esters Isolated from Oxalis triangularis", Biol. Pharm. Bull. 33(7), (2010), 1 July 2010 (2010-07-01), pages 1242-1245, XP055052218, Retrieved from the Internet: URL:https://www.jstage.jst.go.jp/article/bpb/33/7/33_7_1242/_pdf [retrieved on 2013-02-04]
- DATABASE BIOSIS [Online] BIOSCIENCES INFORMATION SERVICE, PHILADELPHIA, PA, US; 1998, AIDA KAZUHIKO ET AL: "Purification and identification of melanogenesis inhibitory factors in miso", XP002691517, Database accession no. PREV199800258959 & NIPPON SHOKUHIN KAGAKU KOGAKU KAISHI, vol. 45, no. 3, 1998, pages 205-209, ISSN: 1341-027X

Remarks:
    The file contains technical information submitted after the application was filed and not included in this specification

## Description

### Field of the invention

The present invention relates to the technical field of cosmetic and pharmaceutical industry, and particularly it relates to a mixture for the inhibition of melanin biosynthesis.

### State of the Art

As known, melanin is the substance which most contributes in determining the pigmentation of human skin. Practically, melanin, or melanins as usually referred to, are biologically occurring black, brown or reddish pigments which confer a characteristic color to the skin.

Melanin is produced by melanocytes, which are specific cells existing at the basal layer of the epidermis. Essentially, when melanocytes are exposed to light and particularly to ultraviolet radiations, they produce melanin through a biochemical process.

The quite complex process of melanin biosynthesis results from oxidation of the amino acid tyrosine, which is a precursor of melanin.

Although melanin plays an important role in the organism, cases are not uncommon in which there is an undesirable skin hyperpigmentation due to factors which can be endogenous or exogenous in nature such as aging, diseases, for example skin diseases, or skin spots due to sun exposure.

Therefore, in these as well as in other cases, there is the need to control the biosynthesis of melanin, and particularly to decrease the biosynthesis thereof or otherwise to obtain a depigmenting effect on the skin.

In this regard, the prior art has provided several compounds which are mainly, but not only, based on the ability of inhibiting the enzymatic activity of tyrosinase, the enzyme which cooperates with oxygen to allow tyrosine to be oxidized into dopaquinone. Other compounds with depigmenting activity facilitate the proteolytic degradation of tyrosinase, whereas still others inhibit the formation thereof.

With regard to tyrosine, there is to be said that its oxidation is a critical and crucial step in melanin biosynthesis as the subsequent steps occur spontaneously in the human body.

The approach to the inhibition of the enzymatic activity of tyrosinase is generally based on a direct inhibition of the oxidation process of the various melanin synthesis intermediates, and it mainly consists in inhibiting its catalytic ability through a typically competitive-type mechanism.

The compounds known as tyrosinase inhibitors belong to the group of polyphenols including flavonols, flavones, flavonones, isoflavonols, chalcones, stilbenes, coumarins as well as kojic acid, glabridin and, generally, licorice derivatives.

Document WO 2007/077260 describes the depigmenting activity of diphenylmethane and derivatives thereof when used in association with various other substances whose depigmenting and UV radiation protective activities are known.

However, the most powerful depigmenting agent known in the prior art is hydroquinone. Hydroquinone acts through a cytotoxic-type mechanism although its action simultaneously results in inhibiting the growth of melanosomes, moreover it also inhibits the formation of dopaquinone and dioxyphenylalanine, another compound obtained from the oxidation of tyrosine by the enzyme tyrosinase.

However, the effectiveness of hydroquinone is always associated with a high toxic and cytotoxic potential which resulted in the prohibition of use thereof in the cosmetic field in many countries, including those of European Union, Japan and South Africa.

Furthermore, in certain European countries including Italy, its use has also been banned in the pharmaceutical field.

Therefore, the prior art describes several compounds capable of inhibiting the production of the skin pigment melanin, however, it is necessary to cope with a not always satisfactory efficacy and with the risk of having unwanted side effects or even toxic and cytotoxic effects, whereas it would be desirable to have depigmenting substances with a high effectiveness, i.e. able to provide a considerable depigmenting action which is greater than that of the known substances, and able to reduce or even eliminate the unwanted side effects as well as - and especially - the toxic and cytotoxic effects.

Document WO 2009/105294 discloses methods and compositions for whitening or brightening skin comprising at least one whitening active contained in association structures.

Document WO 2010/078985 discloses a cosmetic method for controlling browning of the skin induced by UV radiation, consisting in applying, to the surface of the skin, at least one composition comprising: a) at least one depigmenting agent and b) at least one system for screening out both UVA radiation and UVB radiation; c) and optionally at least one keratolytic agent; in which the composition has a critical wavelength of greater than 370 nm.

Document G. Vielhaber ET AL: "4-(1-Phenylethyl) 1,3-Benzenediol: A New, Highly Efficient Lightening agent", International Journal of Cosmetic Science, 2007, 29, 1 January 2007 (2007-01-01), pages 63-66, XP055052000, internet page: URL: http://onlinelibrary.wiley.com/store/10.1111/j.1467-2494.2007.00355_6.x/asset/j.1467-2494.2007.00355_6.x.pdf?v=1 &t=hcn36y4z&s=668fef297fb66ef8f0 77d9262534c6eb6b2979c9, discloses that 4-(1-phenylethyl)-1,3-benzenediol is an highly efficient skin lightening agent that inhibits melanin synthesis.

Document Jae Sung Hwang ET AL: "Disruption of tyrosinase glycosylation by N-acetylglucosamine and its depigmenting effects in guinea pig skin and in human skin", Journal of Dermatological Science 63 (2011), 1 September 2011 (2011-09-01), pages 199-205, XP055052115, internet page URL:http://ac.els-cdn.com/S0923181111001769/1-s2.0-S0923181111001769-main.pdf?_tid=162c13ce-6c8b-11e2-92f2-00000aab0f27&acdnat=1359735720_6f6f568581896de2f81318a71b06 0679, discloses that N-acetylglucosamine reduces melanin in the human skin.

Document Sungran Huh ET AL: "Melanogenesis Inhibitory Effect of Fatty Acid Alkyl esters Isolated from Oxalis triangularis", Biol. Pharm. Bull. 33(7), (2010), 1 July 2010 (2010-07-01), pages 1242-1245, XP055052218, discloses the melanogenesis inhibitory effect of ethyl linoleate.

Document DATABASE BIOSIS [online] BIOSCIENCES INFORMATION SERVICE, PHILADELPHIA, PA, US; 1998, AIDA KAZUHIKO ET AL: "Purification and identification of melanogenesis inhibitory factors in miso", XP002691517, Database accession no. PREV199800258959 ; & NIPPON SHOKUHIN KAGAKU KOGAKU KAISHI, vol 45, no. 3, 1998, pages 205-209, ISSN: 1341-027x, discloses the melanogenesis inhibitory effect of ethyl linoleate.

## Summary of the invention

Subject-matter which is not encompassed by the scope of the claims does not form part of the presently claimed invention.

The technical problem underlying the present invention was to provide a depigmenting product capable to overcome the drawbacks mentioned with reference to the prior art and having the above-cited characteristics.

According to the invention, such a problem has been solved by a mixture comprising acetylglucosamine, 4-(1-phenylethyl)-1,3-benzenediol and ethyl linoleate for use as a cosmetic and/or medicament, particularly for the inhibition of melanin biosynthesis.

Advantageously, the mixture according to the invention can be employed for a cosmetic or pharmaceutical use by external administration, for example by application to the skin or mucous membranes, or by subcutaneous injection, both in case of intact tissue and in case of damaged tissue.

Advantageously, the mixture according to the invention can be used for preventive, ameliorative or curative purposes, in controlling the production of the skin pigment melanin, particularly to decrease melanin production intended as eumelanin and pheomelanin production, e.g. in treating hyperpigmented lesions of the skin, for example but without limitation, lesions which are pathological, inflammatory or infectious in nature, in preventing and/or counteracting signs of skin aging which appear, inter alia, as alterations in uniformity of skin color, wrinkles and loss of skin tone, and in treating sun spots of the skin due to exposure to UV radiation.

The present disclosure has surprisingly shown that, when acetylglucosamine is associated with 4-(1-phenylethyl)-1,3-benzenediol, a derivative of diphenylmethane, it is capable of exerting a remarkable inhibitory action on melanin biosynthesis which is considerably higher than that of each of such two active ingredients when they are used individually.

Particularly, the association of acetylglucosamine with 4-(1-phenylethyl)-1,3-benzenediol is not only significantly more effective than the action individually shown by each of the above compounds, but it is even comparable to that of hydroquinone, the most effective depigmenting agent known in the prior art, while not showing appreciable levels of cytotoxicity as evaluated at the dose used to counteract the process of formation of the skin pigment.

Preferably, the present mixture comprises acetylglucosamine at a concentration by weight in the range between 0.05% and 90%, more preferably between 0.5% and 10%, still more preferably between 2% and 5% based on the total weight of the mixture.

Preferably, the present mixture comprises 4-(1-phenylethyl)-1,3-benzenediol at a concentration by weight in the range between 0.05% and 90%, more preferably between 0.1% and 5.0%, still more preferably between 0.5 and 2% based on the total weight of the mixture. According to the invention, it has been surprisingly found that the effect of the present mixture is further enhanced when ethyl linoleate is present in addition to the above-mentioned active ingredients acetylglucosamine and 4-(1-phenylethyl)-1,3-benzenediol.

Preferably, ethyl linoleate is present in the mixture according to the invention at a concentration by weight in the range between 0.1% and 35%, more preferably between 0.23% and 20%, still more preferably between 2% and 15%, based on the total weight of the mixture.

According to what stated above and still according to the invention, the present mixture is used for the production of a cosmetic and/ or pharmaceutical formulation for the inhibition treatment of melanin biosynthesis, and more generally, it is used for the preparation of a cosmetic and/or medicament for the inhibition of melanin biosynthesis.

Therefore, the invention also provides a cosmetic and/or pharmaceutical formulation comprising the afore said mixture and a physiologically acceptable vehicle or carrier, particularly for the inhibition of melanin biosynthesis.

Here, the term mixture is intended to mean the association of the above active ingredients acetylglucosamine and 4-(1-phenylethyl)-1,3-benzenediol, with ethyl linoleate, in any physical-chemical, solid, liquid or gaseous form such as a blend, a solution, a dispersion, an emulsion, a suspension, an aerosol, a sol, a gel, a foam.

Here, the term formulation is intended to mean any product suitable for the administration of the present mixture through an external or subcutaneous route, for example as an unguent, a cream, an ointment, a paste, a gel, a milk, a lotion, a spray, a solution, an emulsion, a suspension, a soap, and similar forms as typically used in cosmetics and/or pharmacology.

Further characteristics and advantages of the invention will become more apparent upon consideration of the following detailed description of some preferred but not exclusive embodiments which are shown for illustration purposes.

## Detailed description of the invention

A mixture according to the present invention comprises acetylglucosamine, 4-(1-phenylethyl)-1-3-benzenediol (also known as 4-(1-phenylethyl)-1,3-benzenediol or 4-(1-Phenylethyl)benzene-1,3-diol or 4-(1-Phenylethyl)resorcinol or else 4-(alpha-Methylbenzyl)resorcinol) and ethyl linoleate as active ingredients for a cosmetic use and/or as a medicament, particularly for the inhibition of melanin biosynthesis.

Acetylglucosamine is the acetic amide of glucosamine, an amino sugar capable of hindering the glycosylation of the enzyme tyrosinase, thereby inhibiting the maturation of such an enzyme (tyrosinase is a glycoprotein which needs to be glycosylated in order to be active).

4-(1-phenylethyl)-1,3-benzenediol is a derivative of diphenylmethane capable to act as a direct inhibitor of the catalytic activity of tyrosinase and, simultaneously, to act as a preferential substrate over tyrosine (still with reference to tyrosinase) in the oxidation process. 4-(1-phenylethyl)-1,3-benzenediol is characterized, inter alia, by antioxidant and free radical scavenging activities which are features also shown by acetylglucosamine and, therefore, like acetylglucosamine, it is useful in a more general sense to counteract both photo-induced and chronological skin aging.

Surprisingly, the invention has demonstrated that the combined use of the above compounds shows a largely synergistic effect and allows a remarkable inhibitory action on melanin biosynthesis to be obtained at non-cytotoxic levels.

Without wishing to be bound to any scientific theory or demonstration, it is believed that the combined use of acetylglucosamine and 4-(1-phenylethyl)-1,3-benzenediol allows the process of melanin biosynthesis to be controlled through a synchronized mechanism of action in which acetylglucosamine inhibits the process of tyrosinase glycosylation, and thus the bioavailability of this enzyme in the active form, while 4-(1-phenylethyl)-1,3-benzenediol acts to hinder its catalytic activity.

Particularly, obtained experimental data shown that the association of acetylglucosamine and 4-(1-phenylethyl)-1,3-benzenediol can achieve a high depigmenting action which is higher than that achieved by each of these compounds when used individually.

As anticipated above, it is believed that the synergistic effect is determined by the different mechanism of action of the two compounds in modulating the process of melanin biosynthesis.

Specifically, acetylglucosamine is believed to act mainly by inhibiting the glycosylation of tyrosinase and thus the maturation of the enzyme, thereby being able to decrease the bioavailability thereof in its active, glycosylated form, whereas 4-(1-phenylethyl)-1,3-benzenediol is believed to act as an inhibitor of the enzymatic activity and thus of the catalytic activity of tyrosinase.

The two mechanisms lead to an inhibition of melanin biosynthesis which is to be considered not as a simple summation of the intrinsic activities of the two compounds, but rather as an effect, probably obtained through a synchronized biochemical mechanism, which leads to a significant, unexpected decrease of melanin produced by melanocytes.

Indeed, it has been shown that the use of acetylglucosamine alone, when tested at different concentrations of 25 $\mu$g/ml, 50 $\mu$g ml and 75 $\mu$g/ml, cannot cause an appreciable reduction in melanin biosynthesis, showing an average reduction of 8% based on the afore said three concentrations.

On the contrary, when 4-(1-phenylethyl)-1,3-benzenediol is used at a concentration of 10 $\mu$g/ml, it causes a percentage reduction of about 50% in melanin biosynthesis.

Surprisingly, when the two compounds acetylglucosamine and 4-(1-phenylethyl)-1,3-benzenediol are associated and used together at a concentration of 75 $\mu$g/ml and 10 $\mu$g/ml, respectively, the melanogenesis process can be reduced by up to 72% in terms of inhibition of melanin biosynthesis, corresponding to an increase of about 44% compared to 4-(1-phenylethyl)-1,3-benzenediol alone, and of about 25% compared to the mixture of 4-(1-phenylethyl)-1,3-benzenediol and acetylglucosamine. According to the invention, it has been surprisingly shown that, when the present mixture also comprises ethyl linoleate, the inhibition of melanin biosynthesis is further significantly enhanced.

Particularly, it has been shown that a mixture also comprising ethyl linoleate at a concentration of 50 $\mu$g/ml, in

addition to acetylglucosamine and 4-(1-phenylethyl)-1,3-benzenediol at the concentrations indicated above, resulted in an inhibition of melanin biosynthesis close to 82%, with an increase in inhibitory activity on melanin biosynthesis of about 15% as calculated with respect to the inhibitory ability on melanin biosynthesis provided by the association of acetylglucosamine and 4-(1-phenylethyl)-1,3-benzenediol at the afore said concentrations, i.e. 75 $\mu$g/ml and 10 $\mu$g/ml, respectively.

Thus, since it has been shown, inter alia, that the use of ethyl linoleate associated with 4-(1-phenylethyl)-1,3-benzenediol alone at concentrations of 50 $\mu$g/ml and 10 $\mu$g/ml, respectively, causes melanin biosynthesis to be inhibited by about 50%, a result comparable to the percentage of 50% indicated above for 4-(1-phenylethyl)-1,3-benzenediol, then the afore said compound ethyl linoleate can be considered as a specific synergist agent of the mixture according to the invention.

Furthermore, when ethyl linoleate is used in association with acetylglucosamine alone at a concentration of 50 $\mu$g/ml and 75 $\mu$g/ml, respectively, melanin biosynthesis is inhibited by about 15%.

Therefore, in accordance with the reported experimental data, it appears that, when the present mixture comprises essentially acetylglucosamine and 4-(1-phenylethyl)-1,3-benzenediol, a surprising increase in inhibitory ability on melanin production can be achieved, which is about 44% greater than the inhibition obtained with the use of 4-(1-phenylethyl)-1,3-benzenediol alone and yet also significant compared to the sum of the inhibitory effects obtained with the use of the two components individually, said increase being even greater if calculated with respect to the inhibition achieved with the use of acetylglucosamine alone, and moreover, the relative increase in inhibition of melanin is further surprisingly enhanced when a mixture according to the invention is used which also comprises ethyl linoleate, whose ability to inhibit melanin synthesis when used individually is significantly lower than that of the present mixture containing the same ethyl linoleate.

All the compositions have shown to be non-cytotoxic.

According to the same method as described below, the ability of hydroquinone to inhibit melanin biosynthesis when induced at non-cytotoxic concentrations was about 40%.

Therefore, a specific, synergistic mechanism of action can be postulated in which acetylglucosamine causes a decrease in glycosylated - and thus active - tyrosinase while simultaneously promoting a proteasomal digestion which is already stimulated by ethyl linoleate. In this situation, there is also the inhibitory action of 4-(1-phenylethyl)-1,3-benzenediol on the catalytic activity of tyrosinase (that still active and bioavailable).

This mechanism of action, also supported by the experimental data, can be represented by the following schema.

## EXPERIMENTAL SECTION

In order to evaluate the inhibition of melanin biosynthesis by the present mixture, an in vitro experimental model was used which consists of secondary cultures of a murine melanoma named B16. This strain is composed of cells with fibroblastoid morphology which produce melanin (*Source: European Collection of Cell Cultures, ECACC).*

## Sample preparation

For the tests, powders of the active ingredients were dissolved in ethanol and propylene glycol (1:1) at 10% (w/w) followed by dilution in culture medium DMEM high glucose + 10% FBS supplemented with antibiotics (Penicillin 2000 IU/ml, Streptomycin 1000 IU/ml, Gentamicin 10 mg/ml) to final concentrations of:

A) 4-(1-phenylethyl)-1,3-benzenediol: 10 $\mu$g/ml;
B) Acetylglucosamine: 25 $\mu$g/ml, 50 $\mu$g/ml, 75 $\mu$g/ml;
C) Ethyl linoleate: 50 $\mu$g/ml;

D) 4-(1-phenylethyl)-1,3-benzenediol at a concentration of 10 $\mu$g/ml and acetylglucosamine at a concentration of 75 $\mu$g/ml;

E) 4-(1-phenylethyl)-1,3-benzenediol at a concentration of 10 $\mu$g/ml, acetylglucosamine at a concentration of 75 $\mu$g/ml, and ethyl linoleate at a concentration of 75 $\mu$g/ml.

F) hydroquinone (reference) at a concentration of 5 $\mu$g/ml (non-cytotoxic concentration)

### Treatment and exposure

At passage 30 the afore said cells were seeded on 6-well plates for 24 hours at a concentration of 80,000 cells per well. Then, fresh culture medium containing the afore said samples was added. Untreated cells were used as a negative control, whereas cells treated with hydroquinone at a non-cytotoxic concentration of 5 $\mu$g/ml were used as a positive control, as indicated above at point F).

Each sample was tested in duplicate.

After 72 hours of exposure, melanin and intracellular proteins were dosed.

### Melanin dosage

Cells were washed once with phosphate buffered saline (PBS) and then lysed with NaOH 0.1N. The lysate was heated at 60°C for 1 hour to allow melanin to be solubilized.

In order to evaluate the amount of melanin in the cell lysate, a spectrophotometric reading was taken at a wavelength of 405 nm with the use of a colorimeter (Tecan Sunrise Remote Model) equipped with a plate reader.

A standard curve for melanin titrated from 15 to 500 $\mu$g/ml was prepared.

### Measuring of total proteins

The protein dosage was carried out according to the Bradford's method (Bradford M., 1975). At the end of the treatment, cells were washed twice with sterile PBS at 4°C and lysed by treatment with 400 $\mu$l of purified water at 4°C for 15'.

100 $\mu$l of dye reagent 5X was added to the lysate. Then, a standard curve for albumin titrated at 20, 40, 80 and 100 $\mu$g/ml was prepared in a similar manner. Then, 200 $\mu$l of each sample, controls and standards, each in triplicate, was transferred to a 96-well plate.

The reading was taken at 595 nm with a colorimeter (Tecan, Sunrise) equipped with a plate reader.

### Data interpretation

In order to quantify protein and melanin contents, a standard calibration curve for albumin and melanin was constructed, and their concentrations in the samples were calculated by using the curve fit formula and the corresponding absorbances.

Then, averages of individual data were calculated and, for each sample, the percentage reduction of melanin was calculated compared to the negative control:

$$\% \text{ melanin reduction} = \frac{100 - \mu g/ml \text{ melanin} / \mu g/ml \text{ protein content of sample} * 100}{\mu g/ml \text{ melanin} / \mu g/ml \text{ protein content of negative control}}$$

### Formulation examples of the invention

Formulation 1 (reference): Pharmaceutical emulsion for topical use based on 4-(1-phenylethyl)-1,3-benzenediol and acetylglucosamine:

|  | Weight % |
| --- | --- |
| - steareth 2 | 2.00 |
| - steareth 21 | 3.00 |
| - Ppg-15 stearyl ether | 10.00 |
| - stearic acid | 5.00 |
| - butylhydroxytoluene (BHT) | 0.01 |
| - 4-(1-phenylethyl)-1,3-benzenediol | 2.50 |

(continued)

|  | Weight % |
|---|---|
| - Acetylglucosamine | 5.00 |
| - ethyl alcohol | 5.00 |
| - preservatives | q.s. |
| - water | q.s. |

A mixture (said phase A) comprising 2% by weight of Steareth 2, 3% by weight of Steareth 21, 10% by weight of Ppg-15 stearyl ether, 5% by weight of stearic acid, 0.01% by weight of (BHT), and 2.50% by weight of 4-(1-phenylethyl)-1,3-benzenediol was heated to 80°C. This phase A was added with suitable amounts of water and preservatives heated to 75°C; finally, the resulting mixture was added with a phase B consisting of a mixture comprising 5% by weight of acetylglucosamine and 5% of ethyl alcohol heated to 40°C, to obtain formulation 1.

Formulation 2. Emulsion for topical use based on 4-(1-phenylethyl)-1,3-benzenediol, ethyl linoleate and acetylglucosamine:

|  | Weight % |
|---|---|
| - steareth 2 | 2.00 |
| - steareth 21 | 3.00 |
| - Ppg-15 stearyl ether | 10.00 |
| - stearic acid | 5.00 |
| - butylhydroxytoluene (BHT) | 0.01 |
| - 4-(1-phenylethyl)-1,3-benzenediol | 4.50 |
| - Ethyl linoleate | 10.00 |
| - Acetylglucosamine | 5.00 |
| - ethyl alcohol | 5.00 |
| - preservatives | q.s. |
| - water | q.s. |

Formulation 2 was prepared in a manner similar to formulation 1, except that phase A also comprised 10% by weight of Ethyl linoleate.

Therefore, according to the invention, the present mixture comprising the afore said compounds in the different combinations illustrated herein as well as in the different weight ratios indicated above, depending on the desired effect and the chemical-physical form used to apply it to the skin by means of a suitable vehicle or carrier, can be used in the production of cosmetic and/or pharmaceutical formulations intended for use in controlling melanin production and adapted for the treatment of hyperpigmented lesions (such as lentigo solaris, age spots, melasma and chloasma), for rendering uniform the skin color, for counteracting the formation of free radicals and for counteracting or preventing signs of skin aging such as wrinkles and lack of skin tone, and generally for improving the aesthetic conditions of the skin.

The advantages of the present invention, which already appeared evident in the course of the description above, may be summarized by pointing out that it is provided a mixture comprising acetylglucosamine, 4(1-phenylethyl)-1,3-benzenediol and ethyl linoleate use as a cosmetic and/or medicament, and particularly to inhibit melanin biosynthesis, as well as a corresponding cosmetic and/or pharmaceutical formulation comprising the present mixture for the treatment of hyperpigmented lesions of the skin such as, for example but without limitation, lentigo solaris, age spots, melasma and chloasma, for rendering uniform the color of the skin, for counteracting the formation of free radicals and for counteracting or preventing signs of skin aging such as wrinkles and lack of skin tone, and generally for improving the aesthetic conditions of the skin, whose effectiveness in reducing the production of melanin is particularly significant in the absence of cytotoxicity.

Therefore, the present invention represents a substantial, innovative improvement over the prior art known to date, both in terms of effectiveness and in terms of safety of use.

In conclusion, the present mixture, in the different embodiments illustrated and described herein, has surprisingly shown a widely greater - i.e. synergistic - effect both compared to the effect obtained by each component of the mixture when used individually and even compared to the sum of the effects obtained by using each component individually, each case being evaluated at a non-cytotoxic level.

**Claims**

1. Mixture comprising acetylglucosamine, 4-(1-phenylethyl)-1,3-benzenediol and ethyl linoleate for use as a cosmetic and/or medicament.

2. Mixture for use according to claim 1, wherein acetylglucosamine is at a concentration by weight of between 0.05% and 90%, preferably between 0.5% and 10%, more preferably between 2% and 5% based on the total weight of the mixture.

3. Mixture for use according to claim 1 or 2, wherein 4-(1-phenylethyl)-1,3-benzenediol is at a concentration by weight of between 0.05% and 90%, preferably between 0.1% and 5.0%, more preferably between 0.5 and 2% based on the total weight of the mixture.

4. Mixture for use according to any one of the preceding claims, wherein the ethyl linoleate is at a concentration by weight of between 0.1% and 35%, preferably between 0.23% and 20%, more preferably between 2% and 15% based on the total weight of the mixture.

5. Mixture for use according to any one of the preceding claims for the inhibition of melanin biosynthesis.

6. Mixture for use according to any one of the preceding claims for the treatment of hyperpigmented skin lesions including lentigo solaris, age spots (spots of aging), melasma, chloasma.

7. Mixture for use according to any one of the preceding claims for rendering uniform the color of the skin and/or counteracting the formation of free radicals and/or counteracting or preventing signs of skin aging such as wrinkles and lack of skin tone.

8. Cosmetic and/or pharmaceutical formulation comprising a mixture according to any one of the preceding claims and a physiologically acceptable carrier.


**Patentansprüche**

1. Mischung umfassend Acetylglucosamin, 4-(1-Phenylethyl)-1,3-benzendiol und Ethyllinoleat zur Verwendung als Kosmetikum und/oder Arzneimittel.

2. Mischung zur Verwendung nach Anspruch 1, wobei Acetylglucosamin in einer Gewichtskonzentration zwischen 0,05 und 90 Gew.-%, bevorzugt zwischen 0,5 und 10 Gew.-% auf, besonders bevorzugt zwischen 2 und 5 Gew.-%, bezogen auf das Gesamtgewicht der Mischung, vorliegt.

3. Mischung zur Verwendung nach Anspruch 1 oder 2, wobei 4-(1-Phenylethyl)-1,3-benzendiol in einer Gewichtskonzentration zwischen 0,05 und 90 Gew.-%, bevorzugt zwischen 0,1 und 5,0 Gew.-% auf, besonders bevorzugt zwischen 0,5 und 2 Gew.-%, bezogen auf das Gesamtgewicht der Mischung, vorliegt.

4. Mischung zur Verwendung nach einem der vorhergehenden Ansprüche, wobei Ethyllinoleat in einer Gewichtskonzentration zwischen 0,1 und 35 Gew.-%, bevorzugt zwischen 0,23 und 20 Gew.-% auf, besonders bevorzugt zwischen 2 und 15 Gew.-%, bezogen auf das Gesamtgewicht der Mischung, vorliegt.

5. Mischung zur Verwendung nach einem der vorhergehenden Ansprüche zur Hemmung der Melaninbiosynthese.

6. Mischung zur Verwendung nach einem der vorhergehenden Ansprüche zur Behandlung hyperpigmentierter Hautläsionen einschließlich lentigo solaris, age spots (Altersflecken), Melasma, Chloasma.

7. Mischung zur Verwendung nach einem der vorhergehenden Ansprüche zur Vergleichsmäßigung der Hautfarbe und/oder zur Entgegenwirkung der Bildung freier Radikale und/oder zur Entgegenwirkung oder Vorbeugung der Anzeichen einer Hautalterung wie Falten und Verlust des Hauttonus.

8. Kosmetische und/oder pharmazeutische Formulierung enthaltend eine Mischung nach einem der vorhergehenden Ansprüche und einen physiologisch verträglichen Träger.

**Revendications**

1. Mélange comprenant de l'acétylglucosamine, du 4-(1-phényléthyl)-1,3-benzènediol et du linoléate d'éthyle à utiliser comme produit cosmétique et/ou médicament.

2. Mélange à utiliser selon la revendication 1, dans lequel la concentration pondérale de l'acétylglucosamine est comprise entre 0,05% et 90%, de préférence entre 0,5% et 10%, plus préférentiellement entre 2% et 5% par rapport au poids total du mélange.

3. Mélange à utiliser selon la revendication 1 ou 2, dans lequel la concentration pondérale du 4-(1-phényléthyl)-1,3-benzènediol est comprise entre 0,05% et 90%, de préférence entre 0,1% et 5,0%, plus préférentiellement entre 0,5 et 2%, par rapport au poids total du mélange.

4. Mélange à utiliser selon l'une quelconque des revendications précédentes, dans lequel la concentration pondérale du linoléate d'éthyle est comprise entre 0,1% et 35%, de préférence entre 0,23% et 20%, plus préférentiellement entre 2% et 15%, par rapport au poids du mélange.

5. Mélange à utiliser selon l'une quelconque des revendications précédentes pour l'inhibition de la biosynthèse de la mélanine.

6. Mélange à utiliser selon l'une quelconque des revendications précédentes pour le traitement de lésions cutanées hyperpigmentées comprenant lentigo solaris, taches de vieillesse (taches de vieillissement), mélasma, chloasma.

7. Mélange à utiliser selon l'une quelconque des revendications précédentes pour rendre uniforme la couleur de la peau et/ou contrecarrer la formation de radicaux libres et/ou contrecarrer ou prévenir les signes du vieillissement cutané tels que les rides et le manque de tonicité cutanée.

8. Formulation cosmétique et/ou pharmaceutique comprenant un mélange selon l'une quelconque des revendications précédentes et un vecteur physiologiquement acceptable.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2007077260 A
- WO 2009105294 A
- WO 2010078985 A

### Non-patent literature cited in the description

- **G. VIELHABER et al.** 4-(1-Phenylethyl) 1,3-Benzenediol: A New, Highly Efficient Lightening agent. *International Journal of Cosmetic Science,* 01 January 2007, vol. 29, 63-66
- **JAE SUNG HWANG et al.** Disruption of tyrosinase glycosylation by N-acetylglucosamine and its depigmenting effects in guinea pig skin and in human skin. *Journal of Dermatological Science,* 01 September 2011, vol. 63, 199-205
- **SUNGRAN HUH et al.** Melanogenesis Inhibitory Effect of Fatty Acid Alkyl esters Isolated from Oxalis triangularis. *Biol. Pharm. Bull.,* 01 July 2010, vol. 33 (7), 1242-1245
- **AIDA KAZUHIKO et al.** Purification and identification of melanogenesis inhibitory factors in miso. *NIPPON SHOKUHIN KAGAKU KOGAKU KAISHI,* 1998, vol. 45 (3), ISBN 1341-027x, 205-209